# EUROPEAN PATENT SPECIFICATION

(11) **EP 2 046 291 B1**
(45) Date of publication and mention of the grant of the patent: **16.01.2013**
(21) Application number: 07799557.9
(22) Date of filing: 13.07.2007
(51) Int. Cl.: A61K 9/10, A61K 47/26, A61K 47/36, A61K 47/38, A61K 31/167, A61K 31/137, A61K 31/4402, A61K 31/485

(54) **PHARMACEUTICAL SUSPENSIONS COMPRISING PHENYLEPHRINE AND METHOD OF PREPARATION**
PHARMAZEUTISCHE SUSPENSIONEN MIT PHENYLEPHRIN UND ZUBEREITUNGSVERFAHREN
SUSPENSIONS PHARMACEUTIQUES COMPRENANT DE LA PHENYLEPHRINE ET PROCEDE DE PREPARATION

(30) Priority: 13.07.2006 US 457316
(43) Date of publication of application: 15.04.2009
(73) Proprietor: McNeil-PPC, Inc., Skillman, NJ 08558 (US)
(72) Inventor: BUEHLER, Gail K., Ambler, PA 19002 (US); KOCH, Edward A., Mohnton, PA 19540 (US); RECHEN, Dana J., Lansdale, PA 19446 (US)
(74) Representative: Kirsch, Susan Edith
(86) International application number: PCT/US2007/073446
(87) International publication number: WO 2008/008944

(56) References cited:
- EP-A- 0 620 001
- WO-A-03/047502
- WO-A-2004/066978
- WO-A-2006/050302
- GB-A- 829 537
- US-B1- 6 287 597

## Description

### FIELD OF THE INVENTION

This invention relates to liquid pharmaceutical compositions. More particularly, the invention relates to pharmaceutical suspensions containing phenylepherine and at least one substantially water insoluble active agent, e.g., acetaminophen.

### BACKGROUND OF THE INVENTION

Orally administered medicaments or pharmaceuticals are given to the patient in many forms, including solid forms, such as, capsules, caplets, gel caps, or tablets, and liquid forms, such as, emulsions, suspensions, or solutions, e.g., syrups and elixirs. Medicaments administered in solid form are usually intended to be swallowed whole, therefore, the often disagreeable taste of the active agent need not be taken into account in formulating the medicine, except for the provision of means to prevent the taste from being apparent during the short time the medicine is in the mouth. Such means may include the provision of an appropriately thin and quickly dissolving coating on a tablet or caplet or the use of a gelatin capsule form, (the gelatin outer shell of the capsule keeps the active agent inside until the capsule has been swallowed), or simply compressing a tablet firmly so that it will not begin to disintegrate during the short time that it is intended to be in the mouth.

Children, older persons, and many other persons, including disabled or incapacitated patients, have trouble swallowing solid dosage forms of medicine, e.g., whole tablets and even capsules. Therefore, in cases where the dosage to be administered cannot be made into a very small tablet or capsule, it is desirable to provide the medicine either in a chewable solid form or a liquid form. For many patients, including pediatric and geriatric patients, a liquid oral dosage form is preferable over chewable dosage form because of the ready swallowability without chewing of the liquid dosage form.

A common problem associated with liquid dosage forms is the often disagreeable taste of the active agents that manifest during the time that the liquid dosage form is in the mouth prior to swallowing. While suspensions typically offer superior taste masking to other liquid forms, those skilled in the art are aware of the considerable technical difficulties in producing a stable and organoleptically acceptable suspension.

Suspensions are a two-phase system having solid substantially water insoluble active agent particles dispersed throughout liquid medium. A suspension does not encompass emulsions, which are meant to describe liquids suspended within liquid carriers or syrup formulations containing only substantially fully dissolved pharmaceutical active agents.

The challenges of keeping the substantially water insoluble active agent suspended, assuring stability of the substantially water insoluble active agent, and maintaining dose uniformity for a prolonged period of time, have been previously addressed. See for example, U.S. Pat. Nos. 5,409,907, and 5,374,659.

In a pharmaceutical suspension, typically at least one active agent is present substantially in the form of undissolved solid particles, i.e., the substantially water insoluble active agent. However, in any such system, a portion of such active agent may be in the dissolved state. In formulating such systems, it is advantageous to minimize the amount of drug present in the dissolved state. Minimizing the amount of active agent in solution is advantageous for both the taste and chemical and physical stability of the product.

The stability of acetaminophen (N-acetyl para-aminophenol or "APAP") is affected by formulation excipients that are needed to manufacture an acceptable liquid product. Thus, the challenges of manufacturing an acceptable APAP liquid product are many.

In preparing an APAP suspension product, the formulator must ensure that sensory properties are acceptable and the product is acceptable to the user. In this regard, necessary flavors, sweeteners, and consistency modifiers are added to the product. In addition, the formulator must also be aware of the necessary pharmaceutical attributes that must be incorporated into the product. These include: acceptable suspension properties, stability, and microbial properties. All of these requirements complex the challenge of manufacturing an acceptable APAP suspension product.

The present invention is directed to discovery of a stable aqueous APAP suspension with dissolved phenylephrine product that achieves a palatable dosage form for both geriatric and especially pediatric applications.

### SUMMARY OF THE INVENTION

As embodied and fully described herein, the present invention provides a pharmaceutical suspension composition comprising, consisting of and/or consisting essentially of (a) a therapeutically effective amount of a first active agent consisting essentially of a first substantially water insoluble active agent having an average particle size of between about 10 and about 100 microns, (b) a therapeutically effective amount of phenylephrine and pharmaceutically acceptable salts and esters thereof, (c) an effective amount of a non-reducing sweetener comprising sorbitol, (d) an effective amount of water; and (e) an effective amount of a suspending system; wherein the pharmaceutical suspension has a pH of from about 4 to about 6 and is substantially free of a reducing sugar.

Another embodiment of the present invention includes a decongestant suspension, comprising, consisting of, and/or consisting essentially of (a) a therapeutically effective amount of APAP, (b) a therapeutically effective amount phenylephrine, (c) an effective amount of a non-reducing sweetener; (d) an effective amount of water; and (e) an effective amount of a suspending system; wherein the pharmaceutical suspension has a pH of from about 4 to about 6 and is substantially free of a reducing sugar.

A further embodiment of the present invention includes an A decongestant suspension, comprising, consisting of, and/or consisting essentially of by gram per 100 mL of the suspension about 1 to about 15 APAP; about 0.05 to about 2.0 phenylephrine, about 0.1 to about 0.25 xanthan gum; about 0.4 to about 1 microcrystalline cellulose; about 20 to about 70 sorbitol solution; about 1 to about 20 glycerin; about 0.01 to about 1 flavoring; about 20 to about 50 water; about 0.001 to about 0.10 of an antimicrobial preservative selected from the group consisting of butylparaben, methylparaben, propylparaben, and combinations thereof; about 0.003 to about 0.20 citric acid; and about 0.1 to about 0.5 propylene glycol; wherein the APAP suspension has a pH of from about 5 to about 6 and is substantially free of a reducing sugar.

Yet another embodiment of the present invention includes A decongestant suspension, comprising, consisting of, and/or consisting essentially of by gram per 100 mL of the suspension: about 1 to about 15 APAP; about 0.05 to about 2.0 phenylephrine, a pharmaceutical active selected from the group consisting of about 0.1 to about 1 pseudoephedrine HCl, about 0.01 to about 0.07 chloropheniramine maleate, about 0.05 to about 0.5 dextromethorphan HBr, and mixtures thereof; about 0.1 to about 0.25 xanthan gum; about 0.4 to about 1 microcrystalline cellulose; about 20 to about 70 sorbitol solution, about 1 to about 20 glycerin; about 0.01 to about 1 flavoring; about 20 to about 50 water; about 0.001 to about 0.10 of an antimicrobial preservative selected from the group consisting of butylparaben, methylparaben, propylparaben, and combinations thereof; about 0.003 to about 0.20 citric acid; and about 0.1 to about 0.5 propylene glycol; wherein the APAP suspension has a pH of from about 4 to about 6 and is substantially free of a reducing sugar.

It is to be understood that both the foregoing general description and the following detailed description are exemplary, but are not restrictive, of the invention.

### DETAILED DESCRIPTION OF THE INVENTION

As used herein, the term "reducing sugar" means a sugar that can chemically react with a special copper reagent known as Fehlings solution (alkaline solution), whereby the "reducing" sugar will reduce this copper solution to copper oxide (cuprous oxide). Examples of reducing sugars include, but are not limited to, corn syrup, fructose, dextrose and milk sugars.

As used herein, the term "substantially free of a reducing sugar" means less than about 4 g/100 ml of any reducing sugar.

As used herein, the term "APAP" means acetaminophen or N-acetyl para-aminophenol, including, but not limited to, pharmaceutically acceptable salts, esters, isomers, or derivatives thereof.

As used herein, the term "phenylephrine" means benzynemethanol, 3-hydroxy-α-[(methylamino)methyl], and includes, but is not limited to pharmaceutically acceptable salts, esters, isomers or derivatives thereof.

As used herein, the term "substantially water insoluble" refers to compositions that are insoluble, practically insoluble or only slightly soluble in water as defined by U.S. Pharmacopeia, 24th edition. These compositions require at least about 100 parts of solvent per part of said composition, for complete dissolution.

As used herein, a "particle" is a crystal, a granule, an agglomerate, or any undissolved solid material. The particles of the present invention preferably have a median particle size (d50%) of from about 2 to about 150 microns, more preferably from about 10 to about 100 microns.

The present invention provides a suspension system particularly well suited for use in pharmaceutical suspensions. It is the applicants' discovery that a stable and pourable pharmaceutical suspension can be formed having phenylephrine and a substantially water insoluble active agent, e.g., APAP, in suspended form, and optionally further active agents, either at least one additional substantially water insoluble active agent, at least one substantially water soluble active agent, or mixtures thereof, in either suspended, dissolved, or both forms. In certain embodiments the particles are crystals of acetaminophen with a median particle size range (d50%) of from about 10 to about 100 microns or about 10 to about 50 microns.

The invention will now be described specifically in terms of various embodiments. One embodiment includes aqueous suspensions containing phenylephrine and the substantially water insoluble active agent APAP. APAP is a medicament used in both over-the-counter preparations and in prescription drugs for analgesic and antipyretic purposes.

Phenylephrine is generally indicated as a decongestant.

APAP is generally indicated for the temporary relief of minor aches and pains associated with the common cold, headache, toothaches, muscular aches, backache, for minor pain of arthritis, for the pain of menstrual cramps and for the reduction of fever. In certain embodiments, the suspension of the present invention will include APAP in suspended form, together with additional pharmaceutical active agents, which may be present in dissolved or suspended form. Reference will also be made in detail herein to other preferred embodiments of the compositions, processes and methods of the invention.

Suitable additional pharmaceutical active agents include analgesics, anti-inflammatory agents, antiarthritics, anesthetics, antihistamines, antitussives, antibiotics, anti-infective agent, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, additional decongestants, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

In one embodiment of the invention, active agent(s) may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active agent(s) may be selected from analgesics, anti-inflammatories, and antipyretics: e.g., non-steroidal anti-inflammatory drugs (NSAIDs), including propionic acid derivatives: e.g., ibuprofen, naproxen, ketoprofen and the like; acetic acid derivatives: e.g., indomethacin, diclofenac, sulindac, tolmetin, and the like; fenamic acid derivatives: e.g., mefanamic acid, meclofenamic acid, flufenamic acid, and the like; biphenylcarbodylic acid derivatives: e.g., diflunisal, flufenisal, and the like; and oxicams: e.g., piroxicam, sudoxicam, isoxicam, meloxicam, and the like. In a particularly preferred embodiment, the active agent is selected from propionic acid derivative NSAID: e.g., ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another embodiment of the invention, the active agent may be selected from APAP, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active agent(s) may be selected from pseudoephedrine, phenylpropanolamine, chlorpheniramine, dextromethorphan, diphenhydramine, astemizole, terfenadine, fexofenadine, loratadine, desloratidine, doxylamine, doxylamine succinate, chlophedianol, menthol, benzocaine, benzydamine, loratadine, norastemizoie, cetirizine, mixtures thereof and pharmaceutically acceptable salts, esters, isomers, and mixtures thereof.

The pharmaceutical suspension of the present invention may contain at least one additional pharmaceutical active. Such additional pharmaceutical active may be an antihistamine, an antitussive, guafenesin, and a sympathomimetic.

Antihistamine examples include those selected from the group consisting of chloropheniramine maleate, loratadine, desloratidine, terfenadine, astemizole, diphenhydramine hydrochloride and mixtures thereof.

Antitussive examples include those selected from the group consisting of dextromethorphan HBr, diphenhydramine hydrochloride, chlophedianol and mixtures thereof.

Sympathomimetic examples include those selected from the group consisting of pseudoephedrine hydrochloride, phenylpropanolamine, pseudoephedrine and mixtures thereof.

The active agents or ingredients are present in a "unit dose volume" of the aqueous suspension in a therapeutically effective amount, which is an amount that produces the desired therapeutic response upon oral administration and can be readily determined by one skilled in the art. In determining such amounts, the particular active agent being administered, the bioavailability characteristics of the active agent, the dose regime, the age and weight of the patient, and other factors must be considered, as known in the art. As used herein, a "unit dose volume" of the aqueous suspension is a convenient volume for dosing the product to a patient. The dosing directions instruct the patient to take amounts that are multiples of the unit dose volume depending on, e.g., the age or weight of the patient. Typically the unit dose volume of the suspension will contain an amount of active agent(s) that is therapeutically effective for the smallest patient. For example, suitable unit dose volumes may include one teaspoonful (about 5 mL), one tablespoonful (about 15 mL), one dropperful, or one milliliter.

In one embodiment, the aqueous pharmaceutical suspension composition in accordance with the present invention includes from about 0.05% to about 40%, e.g., about 0.05% to about 0.2%, or about 1.6 to about 10%, or about 15 to about 40% weight per volume (w/v) of at least one substantially water insoluble active agent. Amounts of active agents in this range are generally acceptable for taste modifying. It is possible that more than 40% of a substantially water insoluble active agent could be included in the suspension and be sufficiently taste masked for consumer acceptability. Suspensions containing less than 0.05% of pharmaceutical active agents are also possible.

In one embodiment, the aqueous pharmaceutical suspension composition in accordance with the present invention includes and from about 0.02% to about 30%, e.g., about 0.05% to about 0.5%, or about 0.1 to about 0.2% by weight per volume (w/v) of phenylephrine and its associated salts. This is generally acceptable for a therapeutic dose. In one embodiment the phenylephrine is completely dissolved in the suspension.

In another embodiment, the aqueous pharmaceutical suspension composition in accordance with the present invention includes and from about 0.001% to about 30%, e.g.. about 0.001% to about 0.5%, or about 0.001 to about 0.1% by weight per volume (w/v) of phenylephrine and its associated salts. This is generally acceptable for a therapeutic dose. In one embodiment, the phenylephrine is completely dissolved in the suspension.

In one embodiment, in which the first active agent is APAP, the level of active agent in the suspension is from about 80 to about 160 mg per 1.6 mL, or about 5 to about 10% w/v. In another embodiment, in which the first active agent is APAP, the level of active agent in the suspension is from about 80 to about 160 mg per teaspoonful, or about 1.6 to about 3.2 grams per 100 mL, or about 1 to about 4% w/v and having an average particle size of between about 10 and about 100 microns.

It has been found by the present inventor, that the unique combination of phenylephrine and a first active agent with sorbitol and sucrose at a pH of from about 4.0 to 5.0 produces advantageously storage stable and homogeneously dispersed pharmaceutical suspensions.

The suspension of the present invention may also include a taste- masking composition to mask the bitter taste of the actives in the composition, particularly the suspended acetaminophen and the dissolved phenylephrine. Generally, the taste-masking composition contains at least one sweetening agent and at least one flavoring agent. The flavoring agents added to the mixture should be of the type and amount desired for the particular suspension to meet the preferences dictated by the intended consumer of such suspension, e.g., pediatric or adult.

Suitable sweetening agents are non-reducing sugars, polyhydric alcohols, and high intensity sweeteners. Examples of suitable non-reducing sugars include, but are not limited to, the heterodisaccharides sucrose, raffinose, stachyose; the non-reducing homodisaccharide trehalose; and the non-reducing homo-oligosaccharides cyclomaltohexa(...deca)ose (also known as Schardinger dextrins).

The amount of sugar sweetener used in the suspension will vary depending on the degree of sweetening desired for the particular suspension. Generally, the amount of sugar sweetener will be in the range of from 0 to about 110 grams per 100 mL of the suspension. The amount of sugar sweetener can also be in the range of from about 40 grams to about 100 grams per 100 mL of suspension.

Water-soluble high intensity sweeteners also may be employed in place of, or in addition to, sugar sweeteners. Examples of suitable high intensity sweeteners include, but are not limited to, sucralose, aspartame, saccharin, acesulfame, cyclamate, and pharmaceutically acceptable salts and combinations thereof. The amount of high intensity sweetener used in the suspension will vary depending on the degree of sweetening desired for the particular suspension. Generally, the amount of high intensity sweeteners used in the suspension may vary from in the range of 0 to about 5 grams per 100 mL of suspension. In embodiments employing a high intensity sweetener, such as sucralose, aspartame, acesulfame, saccharin, and pharmaceutically acceptable salts thereof, the level of high intensity sweetener is from 0 to about 1 gram per 100 mL of suspension, a useful level is from about 0 to about 0.5 gram per 100 mL of suspension.

The pharmaceutical suspension of the present invention is substantially free of reducing sugars. Monosaccharide reducing sugars unsuitable for use in the present invention include, but are not limited to, glucose, fructose, galactose, ribose, mannose, sorbose, arabinose, and xylose. Reducing oligosaccharides unsuitable for use in the present invention include, but are not limited to, cellobiose, isomaltose, maltose, gentibiose, laminaribiose; maltotriose, maltotetrose, maltopentose, maltohexose. Heterodisaccharides reducing sugars include, but are not limited to, lactose, lactulose, maltulose, melibiose. Reducing sugars are open-chain hydroxy aldehydes and hydroxy ketones, and are readily oxidized to form acids. The basic amino groups of proteins, peptides, and amino acids are readily added via condensation reaction to the carbonyl groups of acyclic (reducing) sugars. The Maillard reaction, a well-known reaction resulting in a brown color formation, procedes via enolization of the resulting glycosylamines. A second type of sugar decomposition reaction, also resulting in formation of a brown color, results from the enolization of hydroxyaldehydes and hydroxyketones at pH less than 4 and at elevated temperatures, followed to dehydration to form furfurals (2-furaldehydes). This type of reaction is sometimes referred to as "caramelization."

Examples of suitable polyhydric alcohols for use as sweeteners in the present invention include, but are not limited to, sorbitol, mannitol, xylitol, erythritol, maltitol, and the like, and combinations thereof. The amount of polyhedric alcohol sweetener used in the suspension will vary depending on the degree of sweetening desired for the particular suspension. Generally, the amount of polyhydric alcohol sweetener will be in the range of from about 0 to about 90 grams per 100 mL of the suspension.

Reducing sugars can also contribute to the degradation of phenylephrine containing compounds.

Suitable flavoring agents include natural and/or artificial flavors such as mints (i.e., peppermint, etc.,), menthol, cinnamon, vanilla, artificial vanilla, chocolate, artificial chocolate, both natural and/or artificial fruit flavors (e.g., cherry, grape, orange, strawberry, etc.) and combinations of two or more thereof. Flavoring agents are often complex mixtures of chemical compounds dissolved or dispersed in an inert medium, such as, propylene glycol. These solutions or dispersions are generally provided as a minor component of the suspension in amounts effective to provide a palatable flavor to the suspension. However, flavoring agents are generally present in the suspension in amounts in the range of from about 0 to about 5 grams per 100 mL of the suspension.

In certain embodiments, optimum masking of the taste of the solid pharmaceutical active in the suspension can be achieved by limiting the amount of water in the suspension available to solubilize the active agent(s). The minimum amount of water also must provide the suspension with a sufficient aqueous base to impart the desired degree of hydration of the suspending agents. In certain such embodiments, taste-masking of bitter pharmaceutical(s) necessitate that the total amount of water contained in the suspension be in the range of from about 25 to about 60, preferably about 30 to about 55, grams per 100 mL of suspension.

The pH of the suspension should be optimized to minimize the solubility and maximize the chemical stability of any unpleasant tasting and hydrolysis susceptible active agent, e.g., APAP. Ideally the pH of the suspension should be as close as possible to 2 pH units above the pKa of a basic active agent, and as close as possible to 2 pH units below the pKa of an acidic active agent. In certain embodiments employing APAP as an active agent, the pH of the suspension should in the range from about 4 to about 6, e.g., from about 4.0 to about 5.9. The suspension can be buffered using pH adjusting agents to maintain the pH of the suspension in the desired pH range. Suitable pH-adjusting agents may be present in the suspension in amounts sufficient to provide the desired degree of pH buffering. The pH-adjusting agents will typically be present in the range of from about 0 to about 1 gram per 100 mL of the pharmaceutical suspension. The pH adjusting agent for an embodiment having as an active agent, and including a suspending system having alkaline polymers, such as, sodium carboxymethylcellulose, may be selected from weak organic acids, such as, citric acid, malic acid, glutamic acid, and the like having acceptable taste characteristics for use in taste masked oral suspensions. Citric acid can be added to the suspension to stabilize the pH of the suspension at between about 3.5 and about 5.5, e.g., from about 4.0 to about 5.0. Citric acid is advantageously added since this pH range (i.e., about . 4.0 to about 5.0) will enhance the stability of the pharmaceutical suspension. A useful pH for the suspension when phenylephrine is a pharmaceutical active used is between about 4.0 and about 5.0 since the phenylephrine will undergo the least degradation in suspension. Antimicrobial preservatives are selected for their activity within this pH range.

Preservatives useful in pharmaceutical suspensions include, but are not limited to, sodium benzoate, potassium sorbate, salts of edetate (also known as salts of ethylenediaminetetraacetic acid, or EDTA, such as, disodium edetate) and parabens (such as, methyl, ethyl, propyl and butyl p-hydroxybenzoic acids esters). The preservatives listed above are exemplary, but each preservative must be evaluated on an empirical basis, in each formulation, to assure the compatibility and efficacy of the preservative. Methods for evaluating the efficacy of preservatives in pharmaceutical formulations are known to those skilled in the art. In a preferred embodiment, sodium benzoate is a particularly useful preservative ingredients to add to a pharmaceutical suspension containing APAP due to superior activity in the particularly preferred pH range of from about 4 to about 5.

Preservatives are generally present in amounts of up to 1 gram per 100 mL of the suspension. Preferably, the preservatives will be present in amounts in the range of from about 0.01 to about 0.5 gram per 100 mL of the suspension. For pharmaceutical suspensions containing APAP, it is useful that the preservative sodium benzoate be present in the range of from about 0.01 to about 0.25 gram per 100 mL of the pharmaceutical suspension. It is most useful that sodium benzoate be present at a concentration of 0.20 gram per 100 mL of the pharmaceutical suspension.

The suspensions also may contain one or more of the following additives: defoaming agents, colorants, e.g., dyes and lakes, surfactants; electrolytes (monovalent cations are currently preferred); and sequestering agents.

In certain optional embodiments, the pharmaceutical suspension of the invention may employ a surfactant for use as a wetting agent to aid in the dispersion of certain hydrophobic active agents. In certain other embodiments, the suspension of the invention may be substantially free of surfactant. In embodiments employing a surfactant, one useful surfactant is a sorbitan oleate ester, particularly, polyoxyethylene sorbitan monooleate also known as polysorbate 80.

The suspensions of the present invention can employ suspending systems as known in the art that include, but are not limited to, at least one thickening component. The thickening component typically includes one or more thickening agents that may be selected from hydrophilic, i.e., water soluble, polymers such as hydrocolloids, swelling or gelling polymers, and the like. In one embodiment, the thickening component combines the attributes of a structuring agent and a swelling agent. In another preferred embodiment, the thickening component combines the attributes of at least two structuring agents, e.g., a primary structuring agent and a secondary structuring agent.

A structuring agent, when introduced into an appropriate aqueous environment, forms an ordered structure, believed to be stabilized by hydrogen bonding and molecular entanglement. Hydrocolloids are a particularly good type of structuring agent. Hydrocolloids are dispersions of particles around which water molecules and solvated ions form a shell-like structure, fluid absorption occurs principally by swelling and enlargement of the structure.

Examples of suitable hydrocolloids include, but are not limited to, alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, karaya, whelan, rhamsan, zooglan, methylan, chitin, cyclodextrin, chitosan, cellulosic polymers such as microcrystalline cellulose, carboxymethylcellulose, and derivatives and combinations thereof. In certain embodiments of the present invention, useful structuring agents may be selected from the hydrocolloids xanthan gum, microcrystalline cellulose, carboxymethylcellulose, and derivatives, co-precipitates, and combinations thereof. In one particularly useful embodiment, the thickening component inlcudes xanthan gum as a primary structuring agent and a co-processed combination of microcrystalline cellulose and carboxymethylcellulose (such as that commercially available from FMC as Avicel RC-591) as a secondary structuring agent.

Xanthan gum is a high molecular weight natural carbohydrate, specifically, a polysaccharide. The xanthan gum suitable for use in the present invention is a high molecular weight polysaccharide produced by Xanthomonas campestris. Techniques and strains for producing this polysaccharide are described in U.S. Pat. Nos. 4,752,580 and 3,485,719 (the disclosures of which are hereby incorporated by reference). The xanthan gum used in the present invention should have a viscosity in a one percent salt solution of from about 1000 to about 1700 cps (mPa-sec). The one percent solution's viscosity should be measured at 25°C with an LV model Brookfield Synchro-Lectric viscometer at 60 rpm, no. 3 spindle. Xanthan gum is available from several commercial suppliers such a RT Vanderbilt Company and CP Kelco. Examples of suitable xanthan gums are Keltrol, Keltrol F, Keltrol T, Keltrol TF, Xantural 180 and Vanzan NF-ST.

In a useful embodiment, the secondary structuring agent used in the present invention is a dried coprecipitated microcrystal of cellulose and sodium carboxymethylcellulose. Sodium carboxymethyl-cellulose is commonly used as a coprecipitate in microcrystalline cellulose. It is particularly useful if the sodium carboxymethylcellulose is included in the range of from about 8 weight percent to about 19 weight percent of the total weight of the coprecipitated microcrystal of cellulose and sodium carboxymethylcellulose. Useful are microcrystalline cellulose products having in the range from about 8 to about 14 weight percent sodium carboxymethylcellulose. These mixtures as described above are commercially available from a variety of sources, including FMC under the trademark Avicel^{®} CL-611, Avicel^{®} RC-581 and Avicel^{®} RC-591.

The thickening component may optionally include a swelling agent, when exposed to an appropriate aqueous environment, expands and may interact with the structuring agent. Pregelatinized starch is a particularly good swelling agent. Pregelatinized starch, also known as "instantized" starch, is precooked so that it swells and begins to thicken instantly when added to cold water. One particularly suitable pregelatinized starch is prepared from modified, stabilized and waxy, maize food starch, and commercially available from National Starch Company as Instant Starch, Ultrasperse M.

In certain embodiments, an optional auxiliary suspending agent is used in the present invention. The auxiliary suspending agent may be selected from the group consisting of hydroxyethylcellulose and a pharmaceutically acceptable salt of carboxymethylcellulose. Suitable pharmaceutically acceptable salts of carboxymethylcellulose include sodium and calcium salts of a polycarboxymethyl ether of cellulose, commercially available as sodium carboxymethylcellulose, USP and calcium carboxymethylcellulose, NF. Sodium carboxymethylcellulose, USP contains between about 6.5 to about 7.5% by weight sodium on a dry basis and is commercially available from Aqualon Co. under the product designation Aqualon. The hydroxyethylcellulose is a partially substituted poly(hydroxyethyl) ether of cellulose. Hydroxycellulose, NF is commercially available from Aqualon Co. under the product designation NATROSOL.

The present invention also provides a process for preparing the aqueous pharmaceutical suspension composition. A useful process includes the following sequential steps:

(a) adding from about 35 to about 40 weight percent of water to achieve suitable volume for mixing;

(b) dispersing from about 0.5 to about 1.0% microcrystalline cellulose and carboxymethylcellulose (co-processed) and from about 0.1 to 0.2% xanthan gum and mixing until hydrated;

(c) adding from about 10 to about 50% sweet polyhydric alcohol, preferably sorbitol, by weight by volume of the total suspension followed by about 5 to about 20% glycerin;

(d) optionally adding from about 20 to about 50% sugar, preferably sucrose, by weight by volume of the total suspension to the dispersion of step (c) in some embodiments and mixing until the ingredients are uniformly dispersed in the mixture;

(e) adding sufficient citric acid anhydrous powder to lower the pH of the solution to between about 4.0 to about5.5 to the mixture of step (d) until the ingredients arc uniformly dispersed throughout the mixture; or in other embodiments , sodium citrate anhydrous powder is added to adjust the pH;

(f) adding a mixture of about 0.1 to about 0.3 % sodium benzoate;

(g) adding about 0.01 to about 0.10% phenylephrine hydrochloride and other suitable water soluble active ingredients; i.e., 0.01 to about 0.03% chlorpheniramine malcate.

(h) adding the substantially insoluble active agent, e.g., from about 2 to about 12% APAP, followed by the flavoring system from about 0.05 to about 0.7% and suitable high intensity sweetener, e.g., from about 0.1 to about 0.5% sucralose; and

(i) adding and mixing sufficient water to the mixture of step (h) to produce a pharmaceutical suspension of 100% desired volume under vacuum.

In useful embodiments of the process an effective amount of preservative, such as, for example, propylparaben and butylparaben, is added to the mixture in step (f) and the suspension in step (i) is subjected to a deaerating step so that the volume of the suspension is adjusted to 100% by addition of water after such deaerating. The flavoring ingredients added to the mixture in step (h) may be of the type and amount desired for the particular suspension to meet the preferences dictated by the intended consumer of such suspension e.g., pediatric or adult. A more detailed example of a useful process of the present invention is provided in the following examples.

Suspension viscosity is measured using a Brookfield LV Viscometer equipped with Spindle #31. Sample from a previously unopened bottle was dispensed into the sample chamber and equilibrated in a water bath to 25°C. After equilibration, sample was stirred at 1.5 rpm and viscosity read after 2 minutes.

Useful viscosity of the suspension of the present invention is from about 1500 to about 7000 centipoise, e.g., not less than 1800 centipoise, or not less than 2300 centipoise when measured according to the above method.

### EXAMPLES

The invention will now be illustrated by examples. The examples are not intended to be limiting of the scope of the present invention but read in conjunction with the detailed and general description above, provide further understanding of the present invention and an outline of a preferred process for preparing the compositions of the invention.

### Example 1 Cold Suspension Formulation

Phenylephrine HCl with Acetaminophen and Chlorpheniramine Malcate

| Ingredient | Amount (%weight/volume) |
|---|---|
| Purified Water USP | 44.0 |
| Microcrystalline Cellulose and Carboxymethylcellulose Sodium NF | 0.70 |
| Xanthan Gum NF | 0.14 |
| Sorbitol Solution USP 70% | 20.0 |
| Glycerin USP | 10.0 |
| Sucrose NF | 45.0 |
| Citric Acid USP Anhydrous | 0.090 |
| Sodium Benzoate NF | 0.20 |
| Chlorpheniramine Maleate | 0.020 |
| Phenylephrine Hydrochloride USP | 0.050 |
| Acetaminophen USP Fine Powder | 3.20 |
| Flavoring agents | 0.12 |
| Dyes | 0.0035 |
| TOTAL | 100 mL* |

| | |
|---|---|
| *Volume is equivalent to a weight of 124.3 grams | |

### PROCESSING DIRECTIONS:

1. To a tared vessel (Precision Stainless Co., 2000-gallon mix tank) equipped with an Admix high shear mixer (Roto-Solver model 400RS300), 3,028 L of United States Pharmacopoeia (USP) 29 purified (deionization, reverse osmosis, distillation, ion exchange or filtration) water was added.

2. 53 kg microcrystalline cellulose and carboxymethyl-cellulose sodium available as Avicel RC-591 from FMC Corporation was dispersed with about 10.6 kg xanthan gum available from CP Kelco Corporation and mixed at 800 RPM (SPEED) until hydrated (approximately 40 minutes).

3. 757 kg of glycerin available from Proctor and Gamble Corporation and 1,514 kg of sorbitol solution available from SPI Polyols as a 70% solution was added while mixing at 300 RPM followed by 3,407 kg of sucrose available from Tate and Lyle Corporation and was mixed at 450 RPM for 30 minutes.

4. 6.81 kg of citric acid anhydrous available from Archers Daniels Midland Corporation followed by 15.14 kg sodium benzoate available from Noveon Kalama, Inc., were added, and 1.514 kg chlorpheniramine maleate available from Kongo Chemical Company and 3.785 kg phenylephrine hydrochloride available from Bochringer Ingelheim were added and mixed at 880 RPM for 30 minutes.

5. 242.2 kg of acetaminophen fine powder grade (i.e., less than 50 µm mean particle size, or you could say d50=14µm whereas our standard powder has d50=32µm) available from Mallincrodt Corporation was added and dispersed into the mixture from Step 4 in the mix tank followed by 0.265 kg of dyes available from Sensient Colors pre-mixed in 20 L of purified water and the flavor system available from Firmenich Corporation and then mixed at 880 RPM for 20 minutes.

6. The suspension was brought to final volume with purified water, and mixed under vacuum at a minimum vacuum level of 4.0 psia (pounds per square inch absolute) for 20 minutes to deacrate (all mix steps mixed under vacuum as well).

The above produced a batch size of approximately 7,570 L or 2000 gallons of product at a pH of 4.5.

### Example 2 Multi-Symptom Cold/Flu Suspension Formulation

### Phenylephrine HCl with Acetaminophen, Dextromethorphan HBr and Chlorpheniramine Maleate

| Ingredient | Amount (%w/v) |
|---|---|
| Purified Water USP | 44 |
| Microcrystalline Cellulose and Carboxymethylcellulose Sodium NF | 0.70 |
| Glycerin USP | 10 |
| Dextromethorphan Hydrobromide USP | 0.10 |
| Xanthan Gum NF | 0.14 |
| Sorbitol Solution USP 70% | 20 |
| Sucrose NF | 45 |
| Citric Acid USP Anhydrous | 0.090 |
| Sodium Benzoate NF | 0.20 |
| Chlorpheniramine Maleate USP | 0.020 |
| Phenylephrine HydrochlorideUSP | 0.050 |
| Acetaminophen USP Fine Powder | 3.2 |
| Flavoring agents | 0.12 |
| Dyes | 0.0035 |
| TOTAL | 100 mL* |

| | |
|---|---|
| *Volume is equivalent to a weight of 124.3 grams | |

### PROCESSING DIRECTIONS:

1. To a tared vessel (Precision Stainless Co., 2000-gallon mix tank) equipped with an Admix high shear mixer (Roto-Solver model 400RS300), 3,028 L of United States Pharmacopoeia (USP) 29 purified (deionization, reverse osmosis, distillation, ion exchange or filtration?) water was added.

2. 53 kg microcrystalline cellulose and carboxymethyl-cellulose sodium available as Avicel RC-591 from FMC Corporation was dispersed and mixed at 880 RPM until hydrated (approximately 20 minutes).

3. 757 kg of glycerin available from Proctor and Gamble Corporation and 7.57 kg of dextromethorphan hydrobromide available from Divi's Corporation were added and mixed at 880 RPM for 20 minutes until dissolved.

4. 10.6 kg ofxanthan gum available from CP Kelco Corporation was dispersed and mixed at 880 RPM until hydrated (approximately 40 minutes).

5. 1514 kg of sorbitol solution available as a 70% solution from SPI Polyols Corporation followed by 3407 kg of sucrose available from Tate and Lyle Corporation were added and mixed at 450 RPM for 30 minutes.

6. 6.81 kg of citric acid anhydrous available from Archer Daniels Midland Corporation followed by 15.15 kg of sodium benzoate available from Noveon Kalama, Inc, were added, and 1.514 kg chlorpheniramine maleate available from Kongo Chemical Company and 3.785 kg phenylephrine hydrochloride available from Boehringer Ingelheim Corporation and mixed at 880 RPM for 30 minutes.

7. 242.2 kg of acetaminophen fine powder grade (i.e., less than 50 µm mean particle size, with a mean particle size about 14µm to about 32µm) available from Mallincrodt Corporation was added and dispersed into the mixture from Step 6 followed by 0.265 kg of dyes available from Sensient Colors premixed in 20 L of purified water as referenced in USP 29 and the flavor system available from Virginia Dare and mixed at 880 RPM for 20 minutes.

8. The suspension was brought to the final volume with purified water, and mixed under vacuum at a minimum vacuum level of 4.0 psia (pounds per square inch absolute) to deaerate (all mix steps mixed under vacuum as well).

The above produces a batch size of approximately 7570 liters or 2000 gallons of product at a pH of 4.5.

### Example 3 Infant Cold Suspension Drops Formulation

### Phenylephrine HCl and Acetaminophen

| Ingredient | Amount (%w/v) |
|---|---|
| Purified Water USP | 38.5 |
| Sorbitol Solution USP 70% | 62 |
| Microcrystalline Cellulose and Carboxymethylcellulose Sodium NF | 0.90 |
| Xanthan Gum NF | 0.18 |
| Glycerin USP | 5.0 |
| Citric Acid USP Anhydrous | 0.07 |
| Sodium Benzoate NF | 0.20 |
| Phenylephrine Hydrochloride USP | 0.15625 |
| Acetaminophen USP Fine Powder | 10 |
| Sucralose liquid concentrate | 0.45 |
| Flavoring Agents | 0.59 |
| Dyes | 0.003 |
| TOTAL | 100 mL |

| | |
|---|---|
| *Volume is equivalent to a weight of 118 grams | |

### PROCESSING DIRECTIONS:

1. To a tared vessel (Precision Stainless Co., 550-gallon mix tank) equipped with an Admix high shear mixer (Roto-Solver model 280RS250), 1325 L of United States Pharmacopoeia (USP) 29 purified (deionization, reverse osmosis, distillation, ion exchange or filtration) water and 530 kg of sorbitol solution as available as a 70% solution from SPI Polyols, Inc., were added for mixing.

2. 17.04 kg of microcrystalline cellulose and carboxymethylcellulose sodium available as Avicel RC-591 from FMC Corporation was added and dispersed into the vessel from Step 1 and 3.41 kg of xanthan gum available from CP Kelco Corpration was added and mixed for 40 minutes at 880 RPM.

3. 95 kg of glycerin available from Proctor and Gamble Corporation was added while mixing at 300 RPM and followed by the adding remaining sorbitol solution.

4. 1.325 kg of citric acid available from Archer Daniels Midland Corporation anhydrous powder was added followed by 3.786 kg of sodium benzoate available from Noveon Kalama, Inc. and 2.956 kg of phenylephrine hydrochloride available from Boehringer Ingelheim corporation and mixed at 880 RPM for 30 minutes.

5. 189.3 kg of acetaminophen fine powder grade (i.e. less than 50 µm mean particle size, or you could say d50=14µm whereas our standard powder has d50=32µm) available from Mallincrodt Corporation was added followed by 8.58 kg of sucralose liquid concentrate available from Tate and Lyle Corporation, 0.0568 kg of dyes available from Sensient Colors premixed in 10 Liters of purified water and flavor system were added and mixed to disperse active.

6. The suspension was brought to final volume with purified water, and mixed under vacuum at a minimum vacuum level of 4.0 psia (pounds per square inch absolute) to deacrate (all mix steps mixed under vacuum as well).

The above produces a batch size of 1893 Liters of drops product at a pH of 4.5.

### Example 4 Infant Cold + Cough Suspension Drops Formulation

Phenylephrine Hydrochloride, Dextromethorphan HBr and Acetaminophen

| Ingredient | Amount (%w/v) |
|---|---|
| Purified Water USP | 38.75 |
| Sorbitol Solution USP 70% | 62 |
| Microcrystalline Cellulose and Carboxymethylcellulose Sodium NF | 0.90 |
| Glycerin USP | 5.0 |
| Dextromethorphan Hydrobromide USP | 0.3125 |
| Xanthan Gum NF | 0.18 |
| Citric Acid USP Anhydrous | 0.07 |
| Sodium Benzoate NF | 0.20 |
| Phenylephrine Hydrochloride USP | 0.15625 |
| Acetaminophen USP Fine Powder | 10 |
| Sucralose liquid concentrate | 0.24 |
| Flavoring Agents | 0.179 |
| Dyes | 0.015 |
| TOTAL | 100mL |

| | |
|---|---|
| *Volume is equivalent to a weight of 118 grams | |

### PROCESSING DIRECTIONS:

1. To a tared vessel (Precision Stainless Co., 550-gallon mix tank) equipped with an Admix high shear mixer (Roto-Solvcr model 280RS250), 1325 L of United States Pharmacopoeia (USP) 29 purified (deionization, reverse osmosis, distillation, ion exchange or filtration) water and 530 kg of sorbitol solution as available as a 70% solution from SPI Polyols, Inc. were added for mixing.

2. 17.04 kg of microcrystalline cellulose and carboxymethylcellulose sodium available as Avicel RC-591 from FMC Corporation was added and dispersed and mixed at 800 RPM for 20 minutes.

3. 95 kg of glycerin available from Proctor and Gamble Corporation was added while mixing at 300 RPM and 5.92 kg of dextromethorphan available from Divi's Laboratories were added while mixing at 600 RPM and the batch mixed at 800 RPM for 20 minutes.

4. 3.407 kg of xanthan gum available from CP Kelco Corporation was added and the batch mixed at 880rpm for 40 minutes.

5. 644kg of the remaining sorbitol solution was added at 300rpm.

6. 1.325 kg of citric acid anhydrous powder available from Archer Daniels Midland Corporation was added followed by 3.786 kg of sodium bcnzoatc available from Noveon Kalama, Inc, and 2.958 kg of phenylephrine hydrochloride available from Boehringer Ingelheim Corporation and mixed at 880 RPM for 30 minutes.

5. 189.3 kg of acetaminophen fine powder grade (i.e. less than 50 µm mean particle size, or a mean particle size of about 14 µm to about 32 µm) available from Mallincrodt Corporation was added followed by 4.54 kg of sucralose liquid concentrate available from Tate and Lyle Corporation, 0.284 kg of dyes available from Sensient Colors premixed in 10 Liters of water and flavor system was added and mixed to disperse active.

6. The suspension was brought to final volume with purified water, and mixed under vacuum at a minimum vacuum level of 4.0 psia (pounds per square inch absolute) to deacrate (all mix steps mixed under vacuum as well).

The above produces a batch size of 1893 Liters of drops product at a pH of 4.5.

### Example 5 PHE Suspensions Stability Results

| Formula | Timepoint | Phenylephrine Assay (%) | Total PhenylephrineD egradants (%) |
|---|---|---|---|
| Cold Suspension | Initial | 99.8 | 0.184 |
| | 3months, 25°C/60% Relative Humidity - Inverted | 100.0 | 0.472 |
| | 3 months, 30°C - Inverted | 99.8 | 0.536 |
| | 3 months, 40°C/Not more than 25% Relative Humidity - Inverted | 96.8 | 1.454 |
| | 6 months 25°C/60% Relative Humidity - Inverted | 99.1 | 0.363 |
| | 6 months 30°C - Inverted | 98.8 | 0.552 |
| | | | |
| Cold Plus Flu Suspension | Initial | 100.0 | 0.236 |
| | 3 months, 25°C/60% Relative Humidity - Inverted | 100.5 | 0.434 |
| | 3 months, 30°C - Inverted | 99.8 | 0.885 |
| | 3 months, 40°C/Not more than 25% Relative Humidity - Inverted | 97.2 | 2.587 |
| | | | |
| Cold Suspension Drops | Initial | 97.8 | 0.077 |
| | 3 months, 25°C/60% Relative Humidity - Inverted | 99.3 | 0.178 |
| | 3 months, 30°C - Inverted | 98.9 | 0.183 |
| | 3 months, 40°C/Not more than 25% Relative Humidity - Inverted | 97.1 | 0.286 |
| | 6 months, 25°C/60% Relative Humidity - Inverted | 98.3 | 0.312 |
| | 6 months, 30°C - Inverted | 98.5 | 0.321 |

The target amount for accelerated stability for phenylephrine is not more than 5% total degradants quantified at 3 months, 40°C and 25% Relative Humidity.

### EXAMPLE 6 PHE Suspensions Assay Method:

Samples were analyzed using an HPLC equipped with a Waters® 717 Autoinjector and a Waters® 486 UV detector set at a wavelength of 214 nm. 5mL samples were dissolved in 50:50:0.1 ::H2O:MeOH:H3PO4. Mobile phase A was prepared using 80% 25mM Sodium Acetate Buffer (pH = 4.6) and 20% Methanol. Mobile phase B was prepared using 50% 25mM Sodium Acetate Buffer (pH = 4.6) and 50% Tetrahydrofuran (THF). Mobile Phase A was run for 0 to 20 minutes at a flow rate of 1 mL/minute, Mobile Phase B was run from 21 to 24 minutes at a flow rate of 2 mL/minute, and Mobile Phase A was run from 25 to 32 minutes at a flow rate of 1 mL/minute. The injection volume was 50 µL with a run time of approximately 32 minutes. A 50 mm cation-exchange column was used. Phenylephrine peaks were quantified at relative retention time between 1.0 and 2.6, relative to the Phenylephrine

## Claims

1. A decongestant suspension, comprising:
(a) a therapeutically effective amount of APAP,
(b) a therapeutically effective amount of phenylephrine and pharmaceutically acceptable salts and esters thereof,
(c) an effective amount of a non-reducing sweetener comprising a combination of sucrose and sorbitol;
(d) an effective amount of water; and
(e) an effective amount of a suspending system;
wherein the pharmaceutical suspension has a pH of from 4 to 5 and is substantially free of a reducing sugar.

2. The suspension of claim 1, wherein the APAP has an average particle size of between about 10 and about 100 microns.

3. The suspension of any preceding claim, wherein the non-reducing sweetener is selected from the group consisting of non-reducing sugars, polyhydric alcohols, high intensity sweeteners, and combinations thereof.

4. The suspension of claim 1, wherein the suspending system consists essentially of about 0.1 to about 0.25 gram per 100 mL of the suspension of xanthan gum and about 0.4 to about 1 gram per 100 mL of the suspension of microcrystalline cellulose.

5. The suspension of claim 1, wherein the suspension further comprises a therapeutically effective amount of a second active agent selected from the group consisting of a second substantially water insoluble active agent, a water-soluble active agent, or mixtures thereof.

6. The suspension of claim 5, wherein the second active agent is selected from the group consisting of an antitussive, an expectorant, an antihistamine, a sympathomimetic, and mixtures thereof.

7. The suspension of claim 6,wherein the at least one second active agent is an antihistamine selected from the group consisting of chloropheniramine maleate, terfenadine, astemizole, diphenhydramine hydrochloride and mixtures thereof.

8. The suspension of claim 1, wherein the suspension further comprises a therapeutically effective amount of a second active agent selected from the group consisting of a second substantially water insoluble active agent, a water-soluble active agent, or mixtures thereof, wherein the second active agent is is an antitussive selected from the group consisting of dextromethorphan HBr, chlorphedianol, diphenhydramine hydrochloride and mixtures thereof.

9. The suspension of claim 6,wherein the second active agent is guaifenesin.

10. The suspension of claim 6, wherein the second active agent is a sympathomimetic selected from the group consisting of pseudoephedrine hydrochloride, phenylpropanolamine and mixtures thereof.

11. The suspension of claim 6, wherein the second active agent comprises pseudoephedrine hydrochloride and chlorpheniramine maleate.

12. The suspension of claim 11, wherein the second active agent further comprises dextromethorphan hydrobromide.

13. The suspension of claim 1, wherein the sweetening agent is selected from the group consisting of xylose, ribose, glucose, mannose, galactose, fructose, dextrose, sucrose, maltose, partially hydrolyzed starch solids, partially hydrolyzed corn syrup solids, sorbitol, xylitol, mannitol, glycerin, aspartame, sucralose, cyclamates, saccharin and mixtures thereof.

14. The suspension of claim 1, comprising about 25 to about 60 grams per 100 mL of the suspension of water.

15. The suspension of claim 1, having a viscosity from about 1500 to about 7000 centipoise at 25°C.

16. The suspension of claim 6, wherein second active agent is substantially dissolved.

17. A decongestant suspension of claim 1, further comprising a taste-masking composition including at least one sweetening agent and at least one flavoring agent.

18. A decongestant suspension, comprising by gram per 100 mL of the suspension:
about 1 to about 15 APAP;
about 0.05 to about 2.0 phenylephrine,
a pharmaceutical active selected from the group consisting of about 0.1 to about 1 pseudoephedrine HCl, about 0.01 to about 0.07 chloropheniramine maleate, about 0.05 to about 0.5 dextromethorphan HBr, and mixtures thereof;
about 0.1 to about 0.25 xanthan gum;
about 0.4 to about 1 microcrystalline cellulose;
about 20 to about 70 sorbitol solution,
about 1 to about 20 glycerin;
about 0.01 to about 1 flavoring;
about 20 to about 50 water;
about 0.001 to about 0.10 of an antimicrobial preservative selected from the group consisting of butylparaben, methylparaben, propylparaben, and
combinations thereof;
about 0.003 to about 0.20 citric acid; and
about 0.1 to about 0.5 propylene glycol;
wherein the APAP suspension has a pH of from 4 to 5 and is substantially free of a reducing sugar.

## Patentansprüche

1. Dekongestionsmittelsuspension, umfassend:
(a) eine therapeutisch wirksame Menge von APAP,
(b) eine therapeutisch wirksame Menge von Phenylephrin und pharmazeutisch unbedenklichen Salzen und Estern davon,
(c) eine wirksame Menge eines nichtreduzierenden Süßungsmittels, umfassend eine Kombination von Saccharose und Sorbit;
(d) eine wirksame Menge Wasser und
(e) eine wirksame Menge eines Suspendiersystems; wobei die pharmazeutische Suspension einen pH-Wert von 4 bis 5 aufweist und weitgehend frei von reduzierendem Zucker ist.

2. Suspension nach Anspruch 1, wobei das APAP eine mittlere Teilchengröße zwischen etwa 10 und etwa 100 Mikron aufweist.

3. Suspension nach einem der vorhergehenden Ansprüche, wobei das nichtreduzierende Süßungsmittel aus der Gruppe bestehend aus nichtreduzierenden Zuckern, mehrwertigen Alkoholen, hochintensiven Süßungsmitteln und Kombinationen davon ausgewählt ist.

4. Suspension nach Anspruch 1, wobei das Suspendiersytem im Wesentlichen aus etwa 0,1 bis etwa 0,25 Gramm Xanthangummi pro 100 mL der Suspension und etwa 0,4 bis etwa 1 Gramm mikrokristalliner Cellulose pro 100 mL der Suspension besteht.

5. Suspension nach Anspruch 1, wobei die Suspension ferner eine therapeutisch wirksame Menge eines zweiten Wirkstoffs aus der Gruppe bestehend aus einem zweiten weitgehend wasserunlöslichen Wirkstoff, einem wasserlöslichen Wirkstoff oder Mischungen davon umfasst.

6. Suspension nach Anspruch 5, wobei der zweite Wirkstoff aus der Gruppe bestehend aus einem Antitussivum, einem Expektorans, einem Antihistaminikum, einem Sympathomimetikum und Mischungen davon ausgewählt ist.

7. Suspension nach Anspruch 6, wobei es sich bei dem mindestens einen zweiten Wirkstoff um ein Antihistaminikum aus der Gruppe bestehend aus Chloropheniraminmaleat, Terfenadin, Astemizol, Diphenhydraminhydrochlorid und Mischungen davon handelt.

8. Suspension nach Anspruch 1, wobei die Suspension ferner eine therapeutisch wirksame Menge eines zweiten Wirkstoffs aus der Gruppe bestehend aus einem zweiten weitgehend wasserunlöslichen Wirkstoff, einem wasserlöslichen Wirkstoff oder Mischungen davon umfasst, wobei es sich bei dem zweiten Wirkstoff um ein Antitussivum aus der Gruppe bestehend aus Dextromethorphan HBr, Chlorphedianol, Diphenhydraminhydrochlorid und Mischungen davon handelt.

9. Suspension nach Anspruch 6, wobei es sich bei dem zweiten Wirkstoff um Guaifenesin handelt.

10. Suspension nach Anspruch 6, wobei es sich bei dem zweiten Wirkstoff um ein Sympathomimetikum aus der Gruppe bestehend aus Pseudoephedrinhydrochlorid, Phenylpropanolamin und Mischungen davon handelt.

11. Suspension nach Anspruch 6, wobei der zweite Wirkstoff Pseudoephedrinhydrochlorid und Chlorpheniraminmaleat umfasst.

12. Suspension nach Anspruch 11, wobei der zweite Wirkstoff ferner Dextromethorphanhydrobromid umfasst.

13. Suspension nach Anspruch 1, wobei das Süßungsmittel aus der Gruppe bestehend aus Xylose, Ribose, Glucose, Mannose, Galactose, Fructose, Dextrose, Saccharose, Maltose, Feststoffanteilen von teilhydrolysierter Stärke, Feststoffanteilen von teilhydrolysiertem Maissirup, Sorbit, Xylit, Mannit, Glycerin, Aspartam, Sucralose, Cyclamaten, Saccharin und Mischungen davon ausgewählt ist.

14. Suspension nach Anspruch 1, umfassend etwa 25 bis etwa 60 Gramm Wasser pro 100 mL der Suspension.

15. Suspension nach Anspruch 1 mit einer Viskosität von etwa 1500 bis etwa 7000 Centipoise bei 25°C.

16. Suspension nach Anspruch 6, wobei der zweite Wirkstoff weitgehend gelöst ist.

17. Dekongestionsmittelsuspension nach Anspruch 1, ferner umfassend eine geschmacksmaskierende Zusammensetzung, die mindestens ein Süßungsmittel und mindestens einen Geschmacksverbesserer enthält.

18. Dekongestionsmittelsuspension, umfassend in Gramm pro 100 mL der Suspension:
etwa 1 bis etwa 15 APAP;
etwa 0,05 bis etwa 2,0 Phenylephrin,
einen pharmazeutischen Wirkstoff aus der Gruppe bestehend aus etwa 0,1 bis etwa 1 Pseudoephedrin HCl, etwa 0,01 bis etwa 0,07 Chloropheniraminmaleat, etwa 0,05 bis etwa 0,5 Dextromethorphan HBr und Mischungen davon;
etwa 0,1 bis etwa 0,25 Xanthangummi;
etwa 0,4 bis etwa 1 mikrokristalline Cellulose;
etwa 20 bis etwa 70 Sorbitlösung;
etwa 1 bis etwa 20 Glycerin;
etwa 0,01 bis etwa 1 Geschmacksverbesserer;
etwa 20 bis etwa 50 Wasser;
etwa 0,001 bis etwa 0,010 eines antimikrobiellen Konservierungsmittels aus der Gruppe bestehend aus Butylparaben, Methylparaben, Propylparaben und
Kombinationen davon;
etwa 0,003 bis etwa 0,20 Citronensäure und
etwa 0,1 bis etwa 0,5 Propylenglykol;
wobei die APAP-Suspension einen pH-Wert von 4 bis 5 aufweist und weitgehend frei von reduzierendem Zucker ist.

## Revendications

1. Suspension de décongestionnant, comprenant :
(a) une quantité thérapeutiquement efficace d'APAP,
(b) une quantité thérapeutiquement efficace de la phényléphrine et des sels et esters pharmaceutiquement acceptables de celle-ci,
(c) une quantité efficace d'un édulcorant non réducteur comprenant une combinaison de saccharose et de sorbitol ;
(d) une quantité efficace d'eau ; et
(e) une quantité efficace d'un système de suspension ;
la suspension pharmaceutique ayant un pH de 4 à 5 et étant sensiblement dépourvue de sucre réducteur.

2. Suspension selon la revendication 1, **caractérisée en ce que** l'APAP a une taille moyenne de particules comprise entre environ 10 et environ 100 microns.

3. Suspension selon l'une ou l'autre des revendications précédentes, **caractérisée en ce que** l'édulcorant non réducteur est choisi dans le groupe constitué de sucres non réducteurs, d'alcools polyhydriques, d'édulcorants de haute intensité, et de combinaisons de ceux-ci.

4. Suspension selon la revendication 1, **caractérisée en ce que** le système de suspension est constitué essentiellement d'environ 0,1 à environ 0,25 gramme par 100 mL de suspension de gomme xanthane et d'environ 0, 4 à environ 1 gramme par 100 mL de suspension de cellulose microcristalline.

5. Suspension selon la revendication 1, **caractérisée en ce que** la suspension comprend en outre une quantité thérapeutiquement efficace d'un deuxième agent actif choisi dans le groupe constitué d'un deuxième agent actif sensiblement insoluble dans l'eau, d'un agent actif soluble dans l'eau, ou de mélanges de ceux-ci.

6. Suspension selon la revendication 5, **caractérisée en ce que** le deuxième agent actif est choisi dans le groupe constitué par un antitussif, un expectorant, un antihistaminique, un sympathomimétique, et des mélanges de ceux-ci.

7. Suspension selon la revendication 6, **caractérisée en ce que** ledit au moins un deuxième agent actif est un antihistaminique choisi dans le groupe constitué par le maléate de chlorophéniramine, la terfénadine, l'astémizole, le chlorhydrate de diphénhydramine et des mélanges de ceux-ci.

8. Suspension selon la revendication 1, **caractérisée en ce que** la suspension comprend en outre une quantité thérapeutiquement efficace d'un deuxième agent actif choisi dans le groupe constitué par un deuxième agent actif sensiblement insoluble dans l'eau, un agent actif soluble dans l'eau, ou des mélanges de ceux-ci, le deuxième agent actif étant un antitussif choisi dans le groupe constitué par le dextrométhorphane HBr, le chlorphédianol, le chlorhydrate de diphénhydramine et des mélanges de ceux-ci.

9. Suspension selon la revendication 6, **caractérisée en ce que** le deuxième agent actif est la guaifénésine.

10. Suspension selon la revendication 6, **caractérisée en ce que** le deuxième agent actif est un sympathomimétique choisi dans le groupe constitué par le chlorhydrate de pseudoéphédrine, la phénylpropanolamine et des mélanges de ceux-ci.

11. Suspension selon la revendication 6, **caractérisée en ce que** le deuxième agent actif comprend le chlorhydrate de pseudoéphédrine et le maléate de chlorphéniramine.

12. Suspension selon la revendication 11, **caractérisée en ce que** le deuxième agent actif comprend en outre le bromhydrate de dextrométhorphane.

13. Suspension selon la revendication 1, **caractérisée en ce que** l'édulcorant est choisi dans le groupe constitué par le xylose, le ribose, le glucose, le mannose, le galactose, le fructose, le dextrose, le saccharose, le maltose, des matières solides d'amidon partiellement hydrolysé, des matières solides de sirop de maïs partiellement hydrolysé, le sorbitol, le xylitol, le mannitol, la glycérine, l'aspartame, le sucralose, les cyclamates, la saccharine et des mélanges de ceux-ci.

14. Suspension selon la revendication 1, comprenant environ 25 à environ 60 grammes par 100 mL de suspension d'eau.

15. Suspension selon la revendication 1, ayant une viscosité d'environ 1500 à environ 7000 centipoises à 25°C.

16. Suspension selon la revendication 6, **caractérisée en ce que** le deuxième agent actif est sensiblement dissous.

17. Suspension de décongestionnant selon la revendication 1, comprenant en outre une composition masquant le goût comportant au moins un agent édulcorant et au moins un agent aromatisant.

18. Suspension de décongestionnant, comprenant en gramme par 100 mL de suspension :
d'environ 1 à environ 15 d'APAP ;
d'environ 0,05 à environ 2,0 de phényléphrine,
un agent actif pharmaceutique choisi dans le groupe constitué d'environ 0,1 à environ 1 de pseudoéphédrine HCl, d'environ 0,01 à environ 0,07 de maléate de chlorphéniramine,
d'environ 0,05 à environ 0,5 de dextrométhorphane HBr, et des mélanges de ceux-ci ;
d'environ 0,1 à environ 0,25 de gomme xanthane ;
d'environ 0,4 à environ 1 de cellulose microcristalline ;
d'environ 20 à environ 70 de solution de sorbitol ;
d'environ 1 à environ 20 de glycérine ;
d'environ 0,01 à environ 1 d'agent aromatisant ;
d'environ 20 à environ 50 d'eau ;
d'environ 0,001 à environ 0,10 d'un agent conservateur antimicrobien choisi dans le groupe constitué par le butylparaben, le méthylparaben,
le propylparaben, et des combinaisons de ceux-ci ;
d'environ 0,003 à environ 0,20 d'acide citrique ;
et
d'environ 0,1 à environ 0,5 de propylèneglycol ;
la suspension d'APAP ayant un pH de 4 à 5 et étant sensiblement dépourvue de sucre réducteur.
